Europäisches Patentamt

European Patent Office

Office européen des brevets

(11) Numéro de publication : **0 456 630 A1**

# (12) DEMANDE DE BREVET EUROPEEN

(21) Numéro de dépôt : 91870076.6

(51) Int. Cl.⁵ : **C12M 1/00**

(22) Date de dépôt : **08.05.91**

(30) Priorité : 08.05.90 BE 9000489

(43) Date de publication de la demande :
**13.11.91 Bulletin 91/46**

(84) Etats contractants désignés :
**BE DE FR IT LU NL**

(71) Demandeur : **S.A. YVAN PAQUE**
**Rue de l'Arbre Courte Joie 48**
**B-4000 Rocourt (BE)**

(72) Inventeur : **Gosselin, Yves**
**16, avenue André Drouart**
**1160 Bruxelles (BE)**

Inventeur : **Thonart, Philippe**
**20, rue de la Houlette**
**5080 La Bruyère (BE)**
Inventeur : **Hubert, Jean-Benoit**
**5, chemin des Ecureuils**
**5170 Profondeville (BE)**
Inventeur : **Gilsoul, Jean-Jacques**
**19, rue Chapel Dieu**
**5030 Gembloux (BE)**
Inventeur : **Jeanfils, Joseph**
**159, rue Bois Gotha**
**4000 Liège (BE)**

(74) Mandataire : **Callewaert, Jean et al**
**Bureau Gevers S.A. rue de Livourne 7 bte 1**
**B-1050 Bruxelles (BE)**

(54) Cuve pour des processus nécessitant un apport de lumière.

(57)    Cuve pour des processus nécessitant un apport de lumière, en particulier des processus biologiques dans un milieu liquide isolé, ladite cuve étant pourvue d'au moins une ouverture à travers laquelle s'étend au moins un conducteur optique en matière synthétique agencé pour émettre en bout de conducteur de la lumière servant à produire au moins un spot lumineux dans ledit milieu. Ladite ouverture donne accès à un espace délimité à l'intérieur de la cuve par une enveloppe dans laquelle s'étend ledit conducteur optique, ladite enveloppe comprenant au moins un organe de transmission optique agencé pour produire ledit spot.

EP 0 456 630 A1

Jouve, 18, rue Saint-Denis, 75001 PARIS

Fig.1

La présente invention est relative à un cuve pour des processus nécessitant un apport de lumière, en particulier des processus biologiques dans un milieu liquide isolé, ladite cuve étant pourvue d'au moins une ouverture dans sa face extérieure à travers laquelle s'étend au moins un conducteur optique en matière synthétique agencé pour émettre en bout de conducteur de la lumière transportée par ce dernier, cette lumière servant à produire au moins un spot lumineux dans ledit milieu.

Une telle cuve est connue du brevet d'Allemagne de l'Est n° 212.265. Dans ce brevet, il est décrit une cuve pour la culture de micro-organismes phototrophe dans un milieu nutritif liquide. Pour la croissance de ces organismes, il est nécessaire que de la lumière, sous forme de spots, y soit introduit par l'intermédiaire de conducteurs optiques. Ces spots lumineux sont formés à l'extrémité des conducteurs optiques qui sont introduits directement dans le milieu par l'intermédiaire de l'ouverture.

Toutefois, la culture de ces micro-organismes nécessite des conditions stériles, ce qui implique que tant le conducteur optique qui est immergé dans le milieu que la cuve même soient stérilisés. Un désavantage des cuves connues est que, lors de la stérilisation des conducteurs optiques, qu'elle soit exécutée individuellement ou en même temps que la cuve, la capacité du conducteur optique pour conduire de la lumière s'amoindrit. On a ainsi constaté que, par exemple, après une stérilisation dans un autoclave à 120°C, les conducteurs optiques fabriqués en matière synthétique devenaient un pe plus opaques et que leur capacité de conduction lumineuse s'amoindrissait et, après quelques stérilisations, ces conducteurs devenaient pratiquement inutilisables.

De plus, on a constaté que le plus gros problème ne se situait pas uniquement au niveau de la haute température de 120°C mais bien au niveau de la combinaison de la haute température et de la vapeur régnant dans la cuve lors de la stérilisation, ce qui avait une influence néfaste sur la capacité de conduction lumineuse du conducteur. L'utilisation de conducteurs optiques fabriqués en fibres de verre n'a pas apporté de solution à ce problème puisque ces conducteurs deviennent également opaques et, de plus, à de telles températures, on a même constaté qu'un certain nombre de fibres de verre se cassaient. L'invention a pour but de remédier à ces problèmes qui surgissent lors de la stérilisation.

A cette fin, une cuve suivant l'invention est caractérisée en ce que ladite ouverture donne accès à un espace délimité à l'intérieur de la cuve par une enveloppe dans laquelle s'étend ledit conducteur optique, ladite enveloppe comprenant au moins un organe de transmission optique agencé pour produire ledit spot à partir de lumière émise en bout de conducteur.

Puisque les conducteurs optiques sont isolés dans la cuve par l'enveloppe, ils n'entrent pas en contact direct avec le milieu liquide. Lorsque l'intérieur de la cuve est stérilisé, par exemple en utilisant de la vapeur à 120°C dans un autoclave, le conducteur optique se chauffera mais n'entrera pas en contact avec la vapeur.

De plus, on a constaté qu'en évitant que le conducteur optique fabriqué en matériau synthétique entre en contact avec de l'eau ou de la vapeur lors de la stérilisation, on peut même le chauffer jusqu'à 120°C sans entraver les capacités conductrices lumineuses.

La présence d'une enveloppe autour du conducteur optique dans une cuve pour la culture de micro-organismes photoautotrophes est toutefois connue en soi par l'article "Photoautotrophic Bioreactor using visible solar rays condensed by Fresnel Lenses and transmitted through optical fibers" publié dans "Biotechnology and Bioengineering Symposium No. 15 (1985)" édité par John Wiley & Sons, Inc. Toutefois, ces enveloppes-là ne sont pas utilisées aux fins de protéger le conducteur optique lors de la stérilisation puisque cette enveloppe même est fabriquée dans une matière qui présente les mêmes problèmes que ceux du conducteur optique, lors de la stérilisation. De plus, dans cet article, il n'est pas mentionné que cette enveloppe puisse servir à des fins de protection du conducteur optique lors de la stérilisation. Le but de cette enveloppe connue est d'éviter que des micro-organismes viennent se loger dans des cavités appliquées dans le manteau du conducteur et viennent ainsi empêcher le passage de la lumière dans le milieu liquide. Il sera de plus clair qu'en utilisant de tels conducteurs, on n'obtient pas de spots lumineux dans le milieu et qu'une grande surface lumineuse doit être présente puisque cette lumière diffuse ne permet pas une pénétration profonde dans le milieu.

Une forme de réalisation particulière d'une cuve suivant l'invention est caractérisée en ce que ledit conducteur est constitué essentiellement de polyméthylacrylate. Le polyméthylacrylate est un bon conducteur optique flexible qui de plus résiste à de hautes températures de par exemple 120°C à condition qu'il n'entre pas en contact avec de la vapeur. De plus, un conducteur optique en polyméthylacrylate a un diamètre relativement restreint comparé à d'autres conducteurs optiques ayant les mêmes capacités conductrices lumineuse.

Dans encore une autre forme de réalisation d'une cuve suivant l'invention, ladite enveloppe est essentiellement constituée par au moins un élément en forme de tube dont l'extrémité débouchant dans la cuve est obturée à l'aide dudit élément de transmission optique. Un élément en forme de tube a l'avantage d'être d'une construction relativement simple qui s'adapte parfaitement à un conducteur optique. De préférence, la dimension dudit espace correspond à peu près à celle du conducteur optique. De cette manière, l'enveloppe ne prend qu'un volume restreint, de telle sorte qu'elle ne vienne pas perturber les mouvements giratoires nécessaires dans le milieu.

D'autres avantages et particularités de l'invention seront décrits dans la description donnée ci-dessous d'une cuve convenant à des procédés nécessitant un apport de lumière. Cette description n'est donnée qu'à titre exemplatif et ne limite en aucune façon la portée de l'invention. Les références se rapportent aux dessins annexés, dans lesquels :

la figure 1 illustre schématiquement une coupe transversale d'une cuve suivant l'invention ;

la figure 2 montre une vue explosée d'un détail de la figure 1.

Dans les différentes figures, les mêmes références se rapportent à de même éléments ou à des éléments analogues.

L'invention concerne une cuve pour des processus nécessitant un apport de lumière. Ces processus peuvent être aussi bien des processus biologiques, biochimiques que chimiques. Par processus nécessitant un apport de lumière, on comprend ici non seulement des processus qui utilisent la lumière en tant que source d'énergie mais également des processus dont la régulation est faite au moyen d'apport de lumière. Un exemple d'un processus nécessitant l'apport de lumière est la croissance de micro-organismes phototrophes ou photoautotrophes, tels que par exemple des algues.

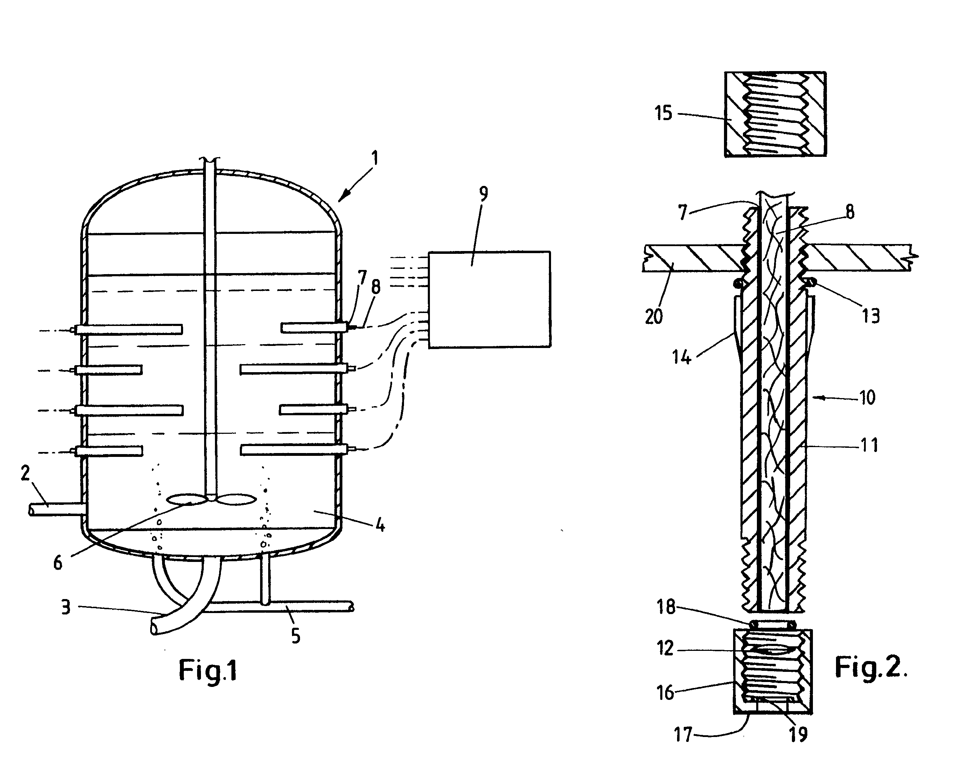

La figure 1 représente schématiquement une cuve suivant l'invention qui convient plus particulièrement à la culture de micro-organismes. La cuve 1 est pourvue d'une entrée 2 et d'une sortie 3 servant à amener et à décharger un milieu liquide 4. La cuve peut également comporter une conduite d'amenée de gaz 5.

La cuve 1 comporte également un élément giratoire 6 pour créer des turbulances dans le milieu 4. Des ouvertures 7 sont prévues dans la paroi de la cuve 1, à travers lesquelles s'étendent des conducteurs optiques 8 en matière synthétique. Une extrémité de ces conducteurs 8 est reliée à une source de lumière 9 qui envoie de la lumière dans les conducteurs optiques. Une autre extrémité des conducteurs optiques 8 se trouve à l'intérieur de la cuve 1 et transmet de la lumière dans le milieu 4. Des conducteurs en polyméthylacrylate ayant un diamètre supérieur à 2 mm, avantageusement entre 7 et 15 mm, mais de préférence d'environ 10mm, conviennent particulièrement bien. Ces conducteurs optiques en polyméthylacrylate peuvent, le cas échéant, être entourés d'une gaîne en Teflon ayant une épaisseur d'environ 1,5 mm.

Dans une cuve 1 suivant l'invention, le conducteur optique 8 n'entre pas en contact direct avec le milieu liquide 4, puisque, tel qu'illustré à la figure 2, le conducteur se trouve à l'intérieur d'une enveloppe 10 qui fait partie de la cuve. Cette enveloppe 10 peut avoir différentes formes mais a, de préférence, la forme d'un tube. Sur la figure 2, cette enveloppe est formée par un élément 11 en forme de tube dont une extrémité est fixée à la paroi extérieure 20 de la cuve 1 et dont l'autre extrémité est obturée à l'aide d'une lentille 12, par exemple une lentille convexe. La liaison entre l'élément 11 en forme de tube et la paroi de la cuve 1 est rendue étanche à l'aide d'un O-ring 13 qui, lors du serrage, vient se caler entre une butée 14, appliquée sur la paroi de cet élément 11, et la paroi interne de la cuve.

Le conducteur optique 8 est fixé à cette enveloppe par l'intermédiaire d'une bague de fixation 15, qui peut être vissée sur l'extrémité de l'élément 11 en forme de tube, qui est en saillie de l'ouverture dans la paroi. Le bout du conducteur optique 8 vient buter contre la lentille 12. Cette lentille forme un organe de transmission optique qui transmet dans le milieu 4 la lumière sortant en bout du conducteur 8. Du fait que le faisceau lumineux est concentré, cette lentille 12 forme, à l'intérieur du milieu 4, un spot lumineux. De préférence, cette lentille est fixée de façon démontable à l'élément 11 en forme de tube. Dans l'exemple de la figure 2, ceci est réalisé à l'aide d'un anneau 16 qui peut être vissé sur l'extrémité de l'élément 11 en forme de tube, lequel anneau 16 est pourvu d'un rebord 17 qui s'étend vers l'intérieur du tube. Un O-ring 18 et un joint au silicone 19 appliqué sur le rebord 17 assurent une bonne étanchéité lors de la fixation de l'anneau 16 à l'extrémité de l'élément 11 en forme de tube.

L'élément optique de transmission ne doit pas être nécessairement formé par une lentille. D'autres éléments capables de transmettre la lumière, tels un prisme ou une plaque en verre, peuvent également être utilisés. L'organe de transmission optique peut également être formé par une combinaison de ces éléments, par exemple par une plaque en verre avec, entre celle-ci et l'extrémité du conducteur optique, une lentille.

Un avantage important d'une cuve suivant l'invention est qu'elle peut être stérilisée sans pour autant porter atteinte aux capacités conductrices de lumière des conducteurs optiques 8. La cuve 1 peut, par exemple, être stérilisée pendant 20 minutes en injectant de la vapeur à 120° dans celle-ci. Puisque l'enveloppe 10 protège les conducteurs optiques contre la vapeur, ces derniers n'entrent donc pas en contact avec cette vapeur. On a constaté plus particulièrement pour des conducteurs en polyméthylacrylate que, à condition qu'ils soient protégés contre la vapeur, ceux-ci ne perdaient pas leur capacité de conduction lumineuse après dix stérilisations a 120°. Des tests ont révélé qu'ils avaient conservé 95% de leur capacité initiale de conduction lumineuse. Par contre lorsqu'ils entraient en contact avec la vapeur durant la stérilisation, ils devenaient opaques.

Un autre avantage d'une cuve suivant l'invention est que les organes de transmission optique forment des spots lumineux dans le milieu. La lumière émise par cet organe dans le milieu pénètre relativement loin dans ce milieu, par exemple à une distance d'environ 10 cm. Grâce à cela, un nombre restreint de spots suffit, par exemple 1 spot par litre. Pour des micro-organismes, tels que des algues, qui circulent dans le milieu, un temps très bref d'exposition à la lumière suffit puisqu'un temps sensiblement plus long est nécessaire pour transformer l'énergie ainsi captée en biomasse. L'utilisation de lentilles permet de concentrer la lumière dans le milieu ou éventuellement de disperser cette lumière lorsqu'une forte concentration de lumière n'est pas nécessaire.

En utilisant des enveloppes dont les dimensions intérieures correspondent approximativement à celles des conducteurs optiques, ces enveloppes ne prennent qu'un espace très restreint dans la cuve. Il reste donc suffisamment de place pour le milieu liquide et une agitation peut donc aisément être effectuée. On pourrait éventuellement encore gagner de la place en divisant le conducteur optique et donc également l'enveloppe, de telle sorte qu'à partir de chaque enveloppe seulement un ou deux spots lumineux sont émis.

Les enveloppes 10 peuvent également servir de contrepales, elles peuvent être constituées par exemple de quatre rangées verticales telles qu'illustrées à la figure 1. Le cas échéant, les quatre enveloppes d'une même rangée peuvent être reliées et former une contre-pale verticale.

Il sera clair que maintes modifications peuvent être apportées à la réalisation décrite ci-dessus, tant pour la forme que pour la composition de la cuve. Le choix des matériaux peut également faire l'objet de multiples variantes.

Les enveloppes peuvent ainsi être formées de n'importe quel matériau, tel que par exemple du métal ou du verre. Eventuellement, les enveloppes peuvent être formées d'un tube en verre obturé à une extrémité.

## Revendications

1. Cuve pour des processus nécessitant un apport de lumière, en particulier des processus biologiques dans un milieu liquide isolé, ladite cuve étant pourvue d'au moins une ouverture dans sa face extérieure à travers laquelle s'étend au moins un conducteur optique en matière synthétique agencé pour émettre en bout de conducteur de la lumière transportée par ce dernier, cette lumière servant à produire au moins un spot lumineux dans ledit milieu, caractérisé en ce que ladite ouverture donne accès à un espace délimité à l'intérieur de la cuve par une enveloppe dans laquelle s'étend ledit conducteur optique, ladite enveloppe comprenant au moins un organe de transmission optique agencé pour produire ledit spot à partir de ladite lumière émise en bout de conducteur.

2. Cuve suivant la revendication 1, caractérisée en ce que ledit conducteur est constitué essentiellement de polyméthyl acrylate.

3. Cuve suivant la revendication 1 ou 2, caractérisée en ce que ledit organe de transmission est formé par une lentille convexe.

4. Cuve suivant l'une quelconque des revendications 1 à 3, caractérisée en ce que ladite enveloppe est essentiellement constituée par au moins un élément en forme de tube dont l'extrémité débouchant dans la cuve est obturée à l'aide dudit élément de transmission optique.

5. Cuve suivant la revendication 4, caractérisée en ce que ledit élément en forme de tube se divise en au moins deux branches.

6. Cuve suivant l'une des revendications 1 à 5, caractérisée en ce que la dimension dudit espace correspond à peu près à celui du conducteur optique.

7. Cuve suivant l'une des revendications 1 à 6, caractérisée en ce que ledit conducteur optique a un diamètre supérieur à 2 mm, avantageusement entre 7 et 15 mm, mais de préférence d'environ 10 mm.

8. Cuve suivant l'une des revendiations 1 à 7, caractérisée en ce que ladite enveloppe est fixée de façon démontable à la cuve.

9. Cuve suivant l'une des revendications 1 à 8, caractérisée en ce que ledit organe de transmission est appliqué de façon démontable sur ladite enveloppe.

10. Enveloppe apte à être utilisée dans une cuve suivant l'une des revendications 1 à 9, caractérisée en ce que ladite enveloppe est connectable à ladite ouverture.

Fig.1

Fig.2.

**Office européen des brevets**

# RAPPORT DE RECHERCHE EUROPEENNE

Numéro de la demande

EP    91 87 0076

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | DE-U-8 703 478 (POHL, P.) 7 Mars 1987<br><br>* page 3, ligne 11 - page 3, ligne 34; figure 2 *<br>--- | 1-2,4,<br>6-7,10 | C12M1/00 |
| Y | PATENT ABSTRACTS OF JAPAN<br>vol. 8, no. 233 (P-309)(1670) 26 Octobre 1974<br>& JP-A-59 111 104 (MITSUBISHI RAYON K.K. ) 27 Juin 1984<br>* le document en entier *<br>--- | 1-2,4,<br>6-7,10 | |
| P,X | US-A-4 952 511 (RADMER, R.J.) 28 Août 1990<br>* colonne 6, ligne 41 - colonne 7, ligne 31; figures 1-4 *<br>--- | 1-4,6,10 | |
| A | EP-A-084 325 (MORI, KEI) 20 Juillet 1983<br>----- | 1,3,4 | |

|  |
|---|
| **DOMAINES TECHNIQUES RECHERCHES (Int. Cl.5)** |
| C12M |

Le présent rapport a été établi pour toutes les revendications

| Lieu de la recherche | Date d'achèvement de la recherche | Examinateur |
|---|---|---|
| LA HAYE | 12 AOUT 1991 | BEVAN S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
--------------------------------------------
& : membre de la même famille, document correspondant

EPO FORM 1503 03.82 (P0402)